# EUROPEAN PATENT APPLICATION

(11) **EP 1 908 454 A1**
(43) Date of publication of application: **09.04.2008**
(21) Application number: 06782125.6
(22) Date of filing: 26.07.2006
(51) Int. Cl.: A61K 8/44, A61Q 19/08

(54) **WRINKLE-PREVENTIVE/AMELIORATING AGENT**

(30) Priority: 26.07.2005 JP 2005215426
(71) Applicant: SHISEIDO COMPANY, LTD., Tokyo 104-8010 (JP)
(72) Inventor: TSUNENAGA, Makoto SHISEIDO RESEARCH CENTER, Yokohama-shi, Kanagawa 224-8558 (JP); IWAKI, Haruhi SHISEIDO RESEARCH CENTER, Yokohama-shi, Kanagawa 224-8558 (JP); IIDA, Toshii SHISEIDO RESEARCH CENTER, Yokohama-shi, Kanagawa 224-8558 (JP); KAMINUMA, Mikiko SHISEIDO RESEARCH CENTER, Yokohama-shi, Kanagawa 224-8558 (JP); SUETSUGU, Masaru SHISEIDO RESEARCH CENTER, Yokohama-shi, Kanagawa 224-8558 (JP); TAKADA, Keiko SHISEIDO RESEARCH CENTER, Yokohama-shi, Kanagawa 224-8558 (JP); INOMATA, Shinji SHISEIDO RESEARCH CENTER, Yokohama-shi, Kanagawa 224-8558 (JP)
(74) Representative: Ulmann, Catherine Claire
(86) International application number: PCT/JP2006/315248
(87) International publication number: WO 2007/013662

(57) **Abstract**

A wrinkle-preventing and -improving composition comprising one or more than one compound selected from the group consisting of α-amino acid derivatives represented by the following general formula (1) and salts thereof: 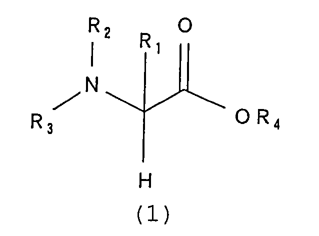 wherein, R₁ represents hydrogen atom, CH₃ group or CH₂OH group, R₂ and R₃ each independently represent hydrogen atom, alkyl group having 1 to 4 carbons, provided that R₂ and R₃ cannot be hydrogen atom at the same time, or R₂ and R₃ together with N atom to which they are bound may form a ring structure having a total carbon number of 4 to 6, in which case said ring structure may optionally contain an oxygen atom as a heteroatom, R₄ represents hydrogen atom, alkyl group having 1 to 18 carbons, wherein when R₁ and R₄ are hydrogen atom, it cannot be that one of R₂ and R₃ is benzyloxycarbonyl group and the other is hydrogen atom.

## Description

### FIELD OF THE INVENTION

The present invention relates to a safe wrinkle-preventing and improving composition that has an effect of preventing wrinkle formation and improving wrinkles.

### BACKGROUND ART

Aging proceeds in all organs of the body. With regard to the skin which is visible, among them, specifically the face on which attention tends to be concentrated, wrinkles including fine wrinkles accompanied with aging are annoying many middle-aged and elderly people, specifically women. Up until now, the need for a wrinkle-improving cosmetic has been pointed out, but since much of the mechanism related to aging and wrinkles was unknown, in respect of conventional cosmetics, biochemical products or synthetic polymer products such as mucopolysaccharides or collagen are blended therein merely to attempt retention of water to improve wrinkle.

However, the above attempt alone cannot fully prevent the aging of the skin or wrinkle formation. In recent years, studies on aging are going on, and as the causes of aging of the skin, macroscopically, aging has been indicated as a major factor, and furthermore drying, oxidation, sunlight (UV rays), etc. have been mentioned as direct factors relating to skin aging. It has become clear that, among them, sunlight (UV rays) is playing an important role in a transformation called photo-aging. The above-mentioned face is the site where photo-aging tends to develop most in the entire body, and it has also become clear that there is a marked decline in the amount of the collagen fiber in the photo-aged skin, which is the most important component in the matrix of dermis. Furthermore, it is suggested that phenomena such as the formation of wrinkles including fine wrinkles and the loss of tautness are closely related to the decline in the amount of collagen fiber. Thus, with regard to wrinkle formation and skin aging, exposure to various skin aging factors, inter alia sunlight, accompanies with reductions of the proliferating activity of fibroblasts, important cells in the dermis, and of the capability of biosynthesizing collagen and the like, which causes a slowdown in the turnover rate of collagen and the like. As a result, the elasticity of the skin is lost, wrinkles are formed, and thus the aging of the skin progresses.

On substances that have an effect of improving wrinkles, amino acids alanine and glycine were known to be effective (Japanese Unexamined Patent Publication (Kokai) No. 11-49628, Japanese Unexamined Patent Publication (Kokai) No. 8-99862). However, alanine and glycine cause acute pain when being applied onto the skin, and thus have difficulty in their use.

Under the above-mentioned circumstances, after extensive and intensive research on a safe substance that has an effect of improving wrinkles without pains even when applied to the skin, the present inventors have found that an α-amino acid derivative and a salt thereof exhibit an excellent effect of improving wrinkles and are safe substances, and thereby have completed the present invention.

### DISCLOSURE OF THE INVENTION

It is an object of the present invention to provide a safe wrinkle-improving composition that has an excellent effect of preventing wrinkle formation or improving wrinkles.

Wrinkles that are formed on the skin have been classified in various ways. As an example of grouping based on shape, Kligman (A.M. Kligman et al.: Br. J. Dermatol., 113: 37-42 (1985)) classified wrinkles into the following classes: linear wrinkles, glyphic wrinkles, and crinkled wrinkles. A representative linear wrinkle is Crow's feet that develop on the sides of the eye. Linear wrinkles and glyphic wrinkles are strongly affected by photo-aging, and the wrinkles are in a relatively deep form. Crinkled wrinkles are wrinkles that develop after the skin surface finely shrinks due to aging, and they are in relatively shallow form and are formed on regions that are not exposed to light. In addition to wrinkles formed due to aging and light, there are wrinkles that develop transiently. They are the so-called facial expression wrinkles, that develop by the shrinkage of mimetic muscles and disappear simultaneously with the restoration of facial expression. However, because the elastic fibers of the dermis and the skin become fragile due to abnormality in dermal elastic fiber or collagen caused by aging, transient wrinkles that disappeared with the cessation of facial movement may become established as permanent wrinkles.

As major causes of shallow wrinkles and deep wrinkles such as described above, there can be mentioned aging, light, and the shrinkage of mimetic muscles, and transformations in the dermis are closely involved. As another factor, drying may be mentioned. When the facial skin is examined, fine wrinkles called skin furrow lines unique to each individual may be observed. When the skin surface becomes dry skin, this line changes into a shape in which this line is highlighted in one direction and thus fine wrinkles develop. As drying leads to the reduced capability of the horny layer to retain water or the loss of flexibility of the skin, it is believed, the resulting strains that could not follow the movement of muscles appeared as fine wrinkles, for which transformations in the dermis are thought to be responsible (Hitoshi Masaki: KOUSHOUKAISI, 25(1): 1647-1649 (1991)).

Fine wrinkles that develop due to drying may be improved by increasing the amount of water in the horny layer with a humectant thereby rendering the horny layer flexible, but for the other wrinkles this is not sufficient and improvement in the constituent components of the dermis is required. The present invention intends to prevent or improve deep wrinkles resulting from the transformations in the dermis.

Thus, the present invention provides a wrinkle-preventing and improving composition comprising one or more than one compound selected from the group consisting of α-amino acid derivatives represented by the following general formula (1) and salts thereof: 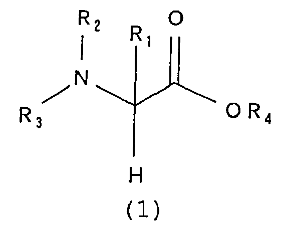 wherein, R₁ represents hydrogen atom, CH₃ group or CH₂OH group,
R₂ and R₃ each independently represent hydrogen atom, alkyl group or alkenyl group having 1 to 4 carbons, acyl group having 2 to 6 carbons, carbamoyl group, amidino group, pyridylcarbonyl group, benzyloxycarbonyl group, cyclohexyl group, cyclohexanecarbonyl group, benzoyl group, benzenesulfonyl group, phenyl group, or benzyl group, provided that R₂ and R₃ cannot be hydrogen atom at the same time, or
R₂ and R₃ together with N atom to which they are bound may form a ring structure having a total carbon number of 4 to 6, in which case the ring structure may optionally contain oxygen atom as a heteroatom,
R₄ represents hydrogen atom, alkyl group or alkenyl group having 1 to 18 carbons, phenyl group, or benzyl group,
wherein the cyclohexyl moiety or the phenyl moiety of R₂, R₃ and R₄, or the ring structure containing N atom formed by R₂ and R₃ may optionally have alkyl group having 1 to 3 carbons, alkoxyl group having 1 to 3 carbons, or hydroxyl group,
or, when R₁ and R₄ are hydrogen atom, it cannot be that one of R₂ and R₃ is benzyloxycarbonyl group and the other is hydrogen atom, nor that one of R₂ and R₃ is amidino group and the other is methyl group.

Alternatively, the present invention provides the above wrinkle-preventing and improving composition wherein the content of a compound selected from the group consisting of α-amino acid derivatives represented by the general formula (1) and salts thereof is 0.001 to 20.0% by weight relative to the total amount of the wrinkle-preventing and improving composition.

Furthermore, the present invention provides the above wrinkle-preventing and improving composition wherein the content of a compound selected from the group consisting of α-amino acid derivatives represented by the general formula (1) and salts thereof is 0.1 to 10.0% by weight relative to the total amount of the wrinkle-preventing and improving composition.

In accordance with the present invention, there can be provided a cosmetic for improving wrinkles having an excellent wrinkle-improving effect.

### BRIEF EXPLANATION OF THE DRAWINGS

Fig. 1 represents the result of a test on the degree of wrinkle-improvement by sarcosine application.
Fig. 2 represents the result of a test on the degree of wrinkle-improvement by benzoyl-DL-serine application.
Fig. 3 represents changes in the ratio of wrinkle area by sarcosine application.
Fig. 4 represents changes in the amount of collagen produced upon sarcosine application, wherein the amount where no sarcosine was added is set as 100.

### BEST MODE FOR CARRYING OUT THE INVENTION

The wrinkle-preventing and improving composition (hereinafter referred to simply as "wrinkle-improving composition") of the present invention contains one or more than one of α-amino acid derivatives represented by the above general formula (1) and salts thereof as active ingredients having a wrinkle-improving effect.

In the formula, R₁ represents hydrogen atom, CH₃ group, or CH₂OH group. When R₁ is hydrogen atom, the derivative corresponds to a glycine derivative, when R₁ is CH₃ group, the derivative corresponds to an alanine derivative, and when R₁ is CH₂OH group, the derivative corresponds to a serine derivative. As both the alanine derivative and the serine derivative have good effect, they may be any of the D form, L form, and DL form (DL mixture), and in respect of the DL form (DL mixture), the blend ratio is not specifically limited.

R₂ and R₃ each independently represent hydrogen atom, alkyl group or alkenyl group having 1 to 4 carbons, acyl group having 2 to 6 carbons, carbamoyl group, amidino group, pyridylcarbonyl group, benzyloxycarbonyl group, cyclohexyl group, cyclohexanecarbonyl group, benzoyl group, benzenesulfonyl group, phenyl group, or benzyl group, provided that R₂ and R₃ cannot be hydrogen atom at the same time.

When one of R₂ and R₃ is alkyl group having 1 to 4 carbons, it may be, as long as it is within this range, linear or branched alkyl group, cyclic alkyl group, or alkyl group containing a cyclic moeity. Specifically, there can be mentioned methyl group, ethyl group, n-propyl group, iso-propyl group, n-butyl group, iso-butyl group, tert-butyl group, 1-methylpropyl group, cyclopropyl group, cyclobutyl group, cyclopropylmethyl group, 1-methylcyclopropyl group, and 2-methylcyclopropyl group.

When one of R₂ and R₃ is alkenyl group having 1 to 4 carbons, it may be, as long as it is within this range, linear or branched alkenyl group, cyclic alkenyl group, or alkenyl group containing a cyclic moiety. Specifically, there can be mentioned ethenyl group, allyl group, 1-propenyl group, 1-butenyl group, 2-butenyl group, 3-butenyl group, 1-methyl-2-propenyl group, 1-methyl-1-propenyl group, 2-methyl-2-propenyl group, 2-methyl-1-propenyl group, butadienyl group, 1-cyclopropenyl group, 2-cyclopropenyl group, 1-cyclobutenyl group, 2-cyclobutenyl group, 1-cyclobutenyl group, 2-cyclobutenyl group, 2-methyl-1-cyclopropenyl group, 3-methyl-1-cyclopropenyl group, 1-methyl-2-cyclopropenyl group, 2-methyl-2-cyclopropenyl group, 2-methylenecyclopropyl group, 3-methylene-1-cyclopropenyl group, cyclopropylmethylenyl group, 2'-cyclopropenylmethylenyl group, cyclobutanedienyl group etc.

When one of R₂ and R₃ is acyl group having 2 to 6 carbons, it may be any group, as long as the number of carbons is within the range of 2 to 6, and specifically there can be mentioned acetyl group, propionyl group, butanoyl group, 2-methylpropionyl group, pentanoyl group, 2-methylbutanoyl group, 3-methylbutanoyl group, 2,2-dimethylpropionyl group, hexanoyl group, 2-methylpentanoyl group, 3-methylpentanoyl group, 2,2-dimethylbutanoyl group, 2,3-dimethylbutanoyl group, 3,3-dimethylbutanoyl group, 2-ethylbutanoyl group, cyclopropropanecarbonyl group, 1-methylcyclopropanecarbonyl group, 2-methylcyclopropanecarbonyl group, 1,2-dimethylcyclopropanecarbonyl group, 2,2-dimethylcyclopropanecarbonyl group, 2,3-dimethylcyclopropanecarbonyl group, cyclobutanecarbonyl group, 1-methylcyclobutanecarbonyl group, 2-methylcyclobutanecarbonyl group, 3-methylcyclobutanecarbonyl group, cycloheptanecarbonyl group, propeonyl group, 2-butenyol group, 3-butenyol group, 2-methylpropeonyl group, 2'-methylenylcyclopropanecarbonyl group, 2'-methylenyl-1'-cyclopropanecarbonyl group, 2'-methylenylcyclopropanemethylcarbonyl group, 2'-methylenyl-1'-cyclopropenemethylcarbonyl group, 2'-methylenylcyclopropanemethylenylcarbonyl group, cyclopropanemethylenylcarbonyl group, 2'-methylcyclopropanemethylenylcarbonyl group, 2'-cyclopropenemethylenylcarbonyl group, 2'-cyclobutanemethylenylcarbonyl group, 2'-cyclobutanemethylenylcarbonyl group, 2'-cyclobutenemethylenylcarbonyl group, 1-cyclobutenecarbonyl group, 2-cyclobutenecarbonyl group, 1,3-cyclobutanedienecarbonyl group, 1'-cyclobutenemethylcarbonyl group, 2'-cyclobutenemethylcarbonyl group, 1-methyl-2-cyclobutenecarbonyl group, 2-methyl-1-cyclobutenecarbonyl group, 2-methyl-2-cyclobutenecarbonyl group, 2-methyl-3-cyclobutenecarbonyl group, 3-methyl-2-cyclobutenecarbonyl group, 1-cyclopentenecarbonyl group, 2-cyclopentenecarbonyl group, 3-cyclopentenecarbonyl group, 1,3-cyclopentadienecarbonyl group, 2,4-cyclopentadienecarbonyl group, and the like.

R₂ and R₃ together with N atom to which they are bound may form a ring structure having a total carbon number of 4 to 6, in which case the ring structure may optionally contain oxygen atom as a heteroatom.

As a specifically formed ring structure, there can be mentioned pyrrolidine, piperidine, azepane (hexamethyleneimine), morpholine and the like.

R₄ represents hydrogen atom, alkyl group or alkenyl group having 1 to 18 carbons, phenyl group, or benzyl group. When R₄ is hydrogen atom, the α-amino acid derivative of the present invention represents an ester compound corresponding to R₄.

As used herein, the cyclohexyl moiety or the phenyl moiety of R₂, R₃ and R₄, or the ring structure containing N atom formed by R₂ and R₃ may optionally have alkyl group having 1 to 3 carbons, alkoxyl group having 1 to 3 carbons, or hydroxyl group.

Alternatively, when R₁ and R₄ are hydrogen atom, it cannot be that one of R₂ and R₃ is benzyloxycarbonyl group and the other is hydrogen atom (benzyloxycarbonyl glycine).

Or, when both of R₁ and R₄ are hydrogen atom, it cannot be that one of R₂ and R₃ is amidine group and the other is methyl group (creatine).

As examples of α-amino acid derivatives, there can be mentioned sarcosine, ethylglycine, propylglycine, butylglycine, N-methyl-L-serine, N-methyl-DL-serine, N-methyl-D-serine, N-ethyl-L-serine, N-ethyl-DL-serine, N-ethyl-D-serine, N-methyl-L-alanine, N-methyl-DL-alanine, N-methyl-D-alanine, N-ethyl-L-alanine, N-ethyl-DL-alanine, N-ethyl-D-alanine, N-carbobenzyloxysarcosine, N-carbobenzyloxy-L-serine, N-carbobenzyloxy-DL-serine, N-carbobenzyloxy-D-serine, N-carbobenzyloxy-L-alanine, N-carbobenzyloxy-DL-alanine, N-carbobenzyloxy-D-alanine, N-acetylglycine, N-acetyl-L-alanine, N-acetyl-DL-alanine, N-acetyl-D-alanine, N-acetyl-L-serine, N-acetyl-DL-serine, N-acetyl-D-serine, N-carbamoylglycine, N-carbamoyl-L-serine, N-carbamoyl-DL-serine, N-carbamoyl-D-serine, N-carbamoyl-L-alanine, N-carbamoyl-DL-alanine, N-carbamoyl-D-alanine, N-amidinoglycine (guanidino acetate), N-amidino-L-serine, N-amidino-DL-serine, N-amidino-D-serine, N-amidino-L-alanine, N-amidino-DL-alanine, N-amidino-D-alanine, N-nicotinoylglycine, N-nicotinoyl-L-serine, N-nicotinoyl-DL-serine, N-nicotinoyl-D-serine, N-nicotinoyl-L-alanine, N-nicotinoyl-DL-alanine, N-nicotinoyl-D-alanine, N-cyclohexylglycine, N-cyclohexyl-DL-serine, N-cyclohexyl-L-serine, N-cyclohexyl-D-serine, N-cyclohexyl-L-alanine, N-cyclohexyl-DL-alanine, N-cyclohexyl-D-alanine, N-benzenesulfonylglycine, N-benzenesulfonyl-L-serine, N-benzenesulfonyl-DL-serine, N-benzenesulfonyl-D-serine, N-benzenesulfonyl-L-alanine, N-benzenesulfonyl-DL-alanine, N-benzenesulfonyl-L-alanine, N-cyclohexanecarbonylglycine, N-cyclohexanecarbonyl-L-alanine, N-cyclohexanecarbonyl-DL-alanine, N-cyclohexanecarbonyl-D-alanine, N-cyclohexanecarbonyl-L-serine, N-cyclohexanecarbonyl-DL-serine, N-cyclohexanecarbonyl-D-serine, N-benzoylglycine, N-benzoyl-L-serine, N-benzoyl-DL-serine, N-benzoyl-L-serine, N-benzoyl-L-alanine, N-benzoyl-DL-alanine, N-benzoyl-D-alanine, N-methoxybenzoylglycine, N-methoxybenzoyl-L-serine, N-methoxybenzoyl-DL-serine, N-methoxybenzoyl-L-serine, N-methoxybenzoyl-L-alanine, N-methoxybenzoyl-DL-alanine, N-methoxybenzoyl-D-alanine, piperidine-1-yl acetate, morpholine-1-yl acetate, pyrrolidine-1-yl acetate, azepane-1-yl acetate, 1H-pyrrole-1-yl acetate, 2-(piperidine-1-yl)propionate, 2-(morpholine-1-yl)propionate, 2-(pyrrolidine-1-yl)propionate, 2-(1H-pyrrole-1-yl)propionate, 2-(azepane-1-yl)propionate, 2-(piperidine-1-yl)-3-hydroxypropionate, 2-(morpholine-1-yl)-3-hydroxypropionate, 2-(pyrrolidine-1-yl)-3-hydroxypropionate, 2-(azepane-1-yl)-3-hydroxypropionate, 2-(1H-pyrrole-1-yl)-3-hydroxypropionate, as well as a product thereof wherein one of these R₂ and R₃ is substituted such as N-methyl product (except N-amidinoglycine), N-ethyl product, N-propyl product, N-butyl product, and N-allyl product, and furthermore a derivative thereof where R₄ is forming an ester such as methyl ester, ethyl ester, propyl ester, hexyl ester, octyl ester, 2-ethylhexyl ester, geranyl ester, decyl ester, dodecyl ester, myristyl ester, lauryl ester, stearyl ester, isostearyl ester, cyclohexyl ester, phenyl ester, and benzyl ester, but not limited to them, and all the derivatives that exhibit a wrinkle-improving effect are useful in the present invention. Preferably, the α-amino acid derivatives of the present invention are sarcosine and ethylglycine, N,N-dimethylglycine, N-methyl-L-serine, N-methyl-DL-serine, N-methyl-D-serine, N-ethyl-L-serine, N-ethyl-DL-serine, N-ethyl-D-serine, N-methyl-L-alanine, N-methyl-DL-alanine, N-methyl-D-alanine, N-ethyl-L-alanine, N-ethyl-DL-alanine, N-ethyl-D-alanine, N-carbobenzyloxysarcosine, N-carbobenzyloxy-L-serine, N-carbobenzyloxy-DL-serine, N-carbobenzyloxy-D-serine, N-carbobenzyloxy-L-alanine, N-carbobenzyloxy-DL-alanine, N-carbobenzyloxy-D-alanine, N-acetylglycine, N-acetylsarcosine, N-acetyl-L-alanine, N-acetyl-DL-alanine, N-acetyl-D-alanine, N-acetyl-L-serine, N-acetyl-DL-serine, N-acetyl-D-serine, N-acetyl-L-serine, N-acetyl-DL-serine, N-acetyl-D-serine, N-carbamoylglycine (hidantoic acid), N-carbamoylsarcosine, N-carbamoyl-L-serine, N-carbamoyl-DL-serine, N-carbamoyl-D-serine, N-carbamoyl-L-alanine, N-carbamoyl-DL-alanine, N-carbamoyl-D-alanine, N-amidinoglycine (guanidino acetate), N-amidino-L-serine, N-amidino-DL-serine, N-amidino-D-serine, N-amidino-L-alanine, N-amidino-DL-alanine, N-amidino-D-alanine, N-nicotinoylglycine, N-nicotinoylsarcosine, N-nicotinoyl-L-serine, N-nicotinoyl-DL-serine, N-nicotinoyl-D-serine, N-nicotinoyl-L-alanine, N-nicotinoyl-DL-alanine, N-nicotinoyl-D-alanine, N-cyclohexylglycine, N-cyclohexylsarcosine, N-cyclohexyl-DL-serine, N-cyclohexyl-L-serine, N-cyclohexyl-D-serine, N-cyclohexyl-L-alanine, N-cyclohexyl-DL-alanine, N-cyclohexyl-D-alanine, N-benzenesulfonylglycine, N-benzenesulfonylsarcosine, N-benzenesulfonyl-L-serine, N-benzenesulfonyl-DL-serine, N-benzenesulfonyl-D-serine, N-benzenesulfonyl-L-alanine, N-benzenesulfonyl-DL-alanine, N-benzenesulfonyl-L-alanine, N-cyclohexanecarbonylglycine, N-cyclohexanecarbonylsarcosine, N-cyclohexanecarbonyl-L-alanine, N-cyclohexanecarbonyl-DL-alanine, N-cyclohexanecarbonyl-D-alanine, N-cyclohexanecarbonyl-L-serine, N-cyclohexanecarbonyl-DL-serine, N-cyclohexanecarbonyl-D-serine, N-benzoylglycine, N-benzoyl-L-serine, N-benzoyl-DL-serine, N-benzoyl-L-serine, N-benzoyl-L-alanine, N-benzoyl-DL-alanine, N-benzoyl-D-alanine, N-benzoylsarcosine, N-methoxybenzoylglycine, N-methoxybenzoyl-L-serine, N-methoxybenzoyl-DL-serine, N-methoxybenzoyl-L-serine, N-methoxybenzoyl-L-alanine, N-methoxybenzoyl-DL-alanine, N-methoxybenzoyl-D-alanine, N-methoxybenzoylsarcosine, piperidine-1-yl acetate, morpholine-1-yl acetate, pyrrolidine-1-yl acetate, azepane-1-yl acetate, 1H-pyrrole-1-yl acetate, 2-(piperidine-1-yl)propionate, 2-(morpholine-1-yl)propionate, 2-(pyrrolidine-1-yl)propionate, 2-(azepane-1-yl)propionate, 2-(1H-pyrrole-1-yl)propionate, 2-(piperidine-1-yl)-3-hydroxypropionate, 2-(morpholine-1-yl)-3-hydroxypropionate, 2-(pyrrolidine-1-yl)-3-hydroxypropionate, 2-(azepane-1-yl)-3-hydroxypropionate, and 2-(1H-pyrrole-1-yl)-3-hydroxypropionate.

The α-amino acid derivatives or salts thereof represented by the above general formula (1) may be commercially available products or those that were synthesized by a known method or a method pursuant to it. Whether they are novel or known in applying to the skin, the fact that they have the wrinkle-preventing and/or improving effect of the present invention is novel.

For example, when the α-amino acid derivative for use in the present invention is sarcosine, sarcosine is a known substance used under the name of N-methylglycine etc. and is known for its use as a raw material for cosmetics (International Cosmetic Ingredient Dictionary and Handbook, Seventh Edition, Volume 2, CTFA (1997)) and as a humectant (Patent No. 3441387), but the wrinkle-preventing and/or improving effect of the present invention is novel.

Also, when the α-amino acid derivative for use in the present invention is N-benzoyl-L-serine, N-benzoyl-L-alanine, N-cyclohexanecarbonyl-L-serine, N-cyclohexanecarbonyl-L-alanine or N-cyclohexanecarbonylglycine, all of them are known substances and are known for their use as perfume precursors (Japanese Unexamined Patent Publication (Kohyo) No. 2002-508307), but the wrinkle-preventing and/or improving effect of the present invention is novel.

Also, when the α-amino acid derivative for use in the present invention is N-benzoylglycine, this compound is a known substance known under the name of hippuric acid and is known for its use as a perfume precursor (Japanese Unexamined Patent Publication (Kohyo) No. 2002-508307), but the wrinkle-preventing and/or improving effect of the present invention is novel.

Also, when the α-amino acid derivative for use in the present invention is N-carbamoylglycine, this compound is a substance known under the name of hydantoinic acid etc. and for its use in the effect of contracting pores (Japanese Unexamined Patent Publication (Kokai) No. 2005-179343), but the wrinkle-preventing and/or improving effect of the present invention is novel.

As the salts of α-amino acid derivatives represented by general formula (1), there can be mentioned, as inorganic salts, hydrochlorides, sulfates, phosphates, hydrobromates, sodium salts, potassium salts, magnesium salts, calcium salts, ammonium salts and the like. As organic salts, there can be mentioned acetates, lactates, maleates, fumarates, tartarates, citrates, methanesulfonates, p-toluenesulfonates, triethanolamine salts, diethanolamine salts, amino acid salts and the like.

In accordance with the present invention, the amount blended of the α-amino acid derivative represented by general formula (1) or a salt thereof is 0.001 to 20.0% by weight, preferably 0.1 to 10.0% by weight relative to the total amount of the wrinkle-improving composition. If the amount is below 0.001% by weight, the wrinkle-improving effect is not sufficient, and, it is believed, even if the amount exceeds 20.0% by weight, further increase in the wrinkle-improving effect cannot be attained.

To the wrinkle-improving composition of the present invention, in addition to the above essential ingredients, there can be mixed, as appropriate, ingredients that can be usually mixed in cosmetics or pharmaceuticals such as liquid lipids and fats, solid lipids and fats, waxes, hydrocarbons, higher fatty acids, higher alcohols, esters, silicones, anionic surfactants, cationic surfactants, amphoteric surfactants, nonionic surfactants, humectants, water-soluble polymer compounds, thickening agents, coating agents, UV ray-absorbing agents, metal ion sequestering agents, lower alcohols, polyhydric alcohols, sugars, amino acids, organic amines, pH-adjusting agents, skin nutrient preparations, vitamins, antioxidants, perfumes, powders, colorants, water and the like. There can also be mixed, as appropriate, L-ascorbic acid ester derivatives and salts thereof such as L-ascorbic acid and salts thereof, L-ascorbic acid phosphates, and L-ascorbic acid sulfates, L-ascorbic acid glycosides and salts thereof such as L-ascorbic acid glucosides, alkoxy salicylic acids and salts thereof such as 4-methoxysalicylic acids and salts thereof, hydroquinone glycosides and salts thereof such as hydroquinone β-D-glucose and hydroquinone α-D-glucose, tranexamic acid and tranexamic acid derivatives such as tranexamic acid methylamide hydrochlorides, resorcin derivatives such as 4-n-butylresorcin, kojic acid, ellagic acid, linoleic acid, camomile extracts, retinoic acid, retinol, retinol acetates, retinol palmitates, glycyrrhizinic acid and derivatives thereof and the like.

The dosage form of the wrinkle-improving agents of the present invention is not specifically limited, and may take any dosage form such as a solution, a solubilzied product, an emulsion, a powder dispersion, a water-oil bilayer, a water-oil-powder trilayer, an ointment, a gel, and an aerosol. The form of usage is not specifically limited, either, and may take any form of a skin lotion, a milky lotion, a cream, an essense, a gelly, a gel, an ointment, a pack, a mask, and a foundation.

The wrinkle-improving composition of the present invention may be used in cosmetic methods for preventing wrinkle formation and/or alleviating or dissolving formed wrinkles by applying to the skin. The administration method and dosage of the wrinkle-improving composition of the present invention in such cosmetic methods are not specifically limited, either, and may be determined as appropriate depending on the dosage form and the state of wrinkles on the skin to be treated, and typically an appropriate amount, for example 0.1 ml to 1 ml per square cm, may be rubbed directly into the skin for a few times, for example once to five times, per day, or after impregnating the appropriate amount into gauze etc., it may be attached to the skin.

### EXAMPLES

The present invention will now be explained in further detail with reference to Examples. It should be noted, however, that the present invention is not limited to these examples.

### Wrinkle-improving test on hairless mice (1)

Mice used are Hr-1 (Skh-1) hairless mice (HOSHINO JIKKEN DOBUTSU (Hoshino experimental animals); 6 to 10 week-old), and wrinkles were formed by modifying a method by Schwarz (Haratake A. et al., J. Invest. Dermatol. 108:769-775, 1997) according to a method (Naganumaa M. et al., Dermatol. Sci. 25:29-35, 2001, Schwartz E., J. Invest. Dermatol. 91:158-161, 1988) of repeating UVB irradiation. Thus, UVB (light source: Toshiba FL-20 SE fluorescent lamp manufactured by Toshiba Electric) was irradiated on the back three times per week for 10 weeks. The dose irradiated was 36 mJ/cm²/irradiation at the start, and gradually increased from week two and after to 216 mJ/cm²/irradiation on week 10. The total dose was 4.6 mJ/cm². The dose of the UV ray used was a value measured using the UVRADIOMETER (UVR-305/365D(II), TOPCON). After UV irradiation was over, the back of mice was photographed, and the degree of wrinkle formation was scored by modifying the method of Bissett et al. (Bissett DL. et al., Photochemistry and Photobiology 46:367-378, 1987) according to the judgment criteria shown in the following Table 1. To only the mice that had wrinkles of a score of 7 or over, the following solution was applied. Scoring of wrinkles was individually carried out by three measurers, and the score was decided after consultation.

**Table 1**

| Score: | Judgment criteria |
|---|---|
| 0 : | No wrinkles are observed. |
| 1 : | Wrinkles are shallower, shorter or smaller in number than those of score 2. |
| 2 : | Shallow wrinkles are observed. |
| 3 : | Wrinkles are deeper or longer than those of score 2, and shallower, shorter or smaller in number than those of score 4. |
| 4 : | Shallow wrinkles are observed throughout. |
| 5 : | Wrinkles are deeper or longer than those of score 4, and shallower or shorter than those of score 6. |
| 6 : | Deep and long wrinkles are observed. |
| 7 : | Wrinkles deeper and longer than those of score 6 are increased. Shallower or shorter than those of score8. |
| 8 : | Deep and long wrinkles are observed throughout. |

The hairless mice that had wrinkles of a score of 7 or over were divided into two groups of five animals per group so that the score of each group is the same, and then to the entire skin of the back of the mice in each group, 100 µl each of the composition of Comparative Example 1 and the composition of Example 1 having the following composition was applied once per day, five times per week, for six consecutive weeks.

### Example 1 composition

| | |
|---|---|
| Sarcosine | 1.0% by weight |
| Alcohol for cosmetics | 50.0 |
| Purified water | the balance |

### Comparative Example 1 composition

| | |
|---|---|
| Alcohol for cosmetics | 50.0% by weight |
| Purified water | Balance |

After the application was over, the back of the mice was photographed, and the degree of wrinkle formation was individually scored according to the judgment criteria shown in the above Table 1 by three measurers with the group name of the animal being blind to them, and the score was decided after consultation.

The degree of improving wrinkles in mice was determined as follows. Thus, the following equation was used in the calculation:
"wrinkle-improving degree" = "score before solution application" - "score after solution application for 6 weeks"

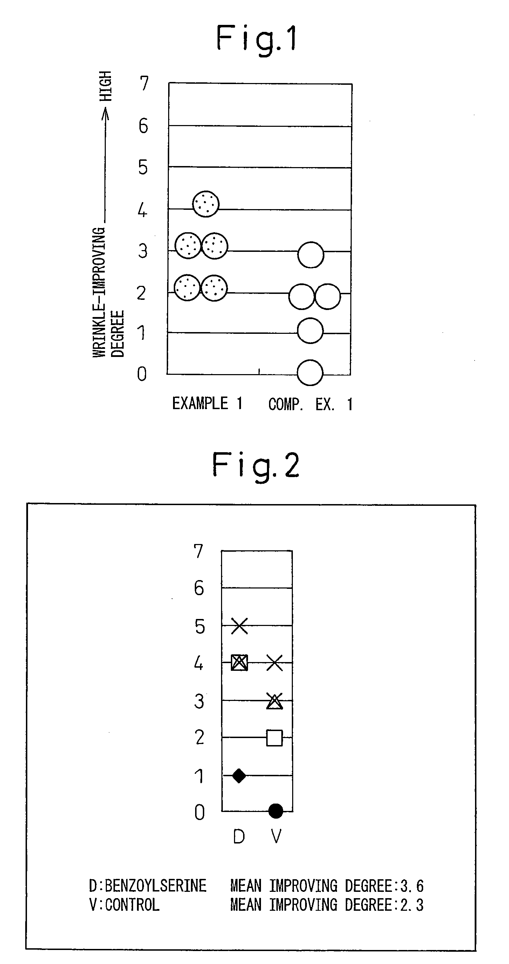

The degree of improving wrinkles obtained by the above equation is shown in Fig. 1.

As can be seen from Fig. 1, reduction in wrinkles was markedly promoted in the Example 1 application group (mean score: 2.8) of the present invention relative to the Comparative Example 1 application group (mean score: 1.6). This confirmed that the sarcosine solution has an effect of improving wrinkles of the skin formed by UV rays.

### Wrinkle-improving test on hairless mice (2)

Using the following sample, a wrinkle-improving test was conducted in a manner similar to that described above. Comparative Example 1 was the same as the one described above.

### Example 2 composition

| | |
|---|---|
| Benzoyl-DL-serine | 3.0% by weight |
| Cosmetic alcohol | 50.0 |
| Purified water | Balance |

The result of the wrinkle-improving degree calculated as in Fig. 2 is shown.

As can be seen from Fig. 2, reduction in wrinkles was markedly promoted in the Example 2 application group (mean score: 3.6) of the present invention relative to the Comparative Example 1 application group (mean score: 2.3). This confirmed that the benzoyl-DL-serine solution has an effect of improving wrinkles of the skin formed by UV rays.

### Wrinkle-improving test on hairless mice (3)

Using the following sample, a wrinkle-improving test was conducted in a manner similar to that described above. Comparative Example 1 was the same as the one described above.

### Example 3 composition

| | |
|---|---|
| Benzyloxycarbonyl-L-serine | 3.0% by weight |
| Cosmetic alcohol | 50.0 |
| Purified water | Balance |

The result was that Example 3 application group of the present invention showed a mean score of 3.5, whereas Comparative Example 1 application group showed a mean score of 2.4. As can be seen from the result, reduction in wrinkles was markedly promoted. This confirmed that the benzyloxycarbonyl-L-serine solution has an effect of improving wrinkles of the skin formed by UV rays.

### Wrinkle-improving test on hairless mice (4)

Using the following sample, a wrinkle-improving test was conducted in a manner similar to that described above. Comparative Example 1 was the same as the one described above.

### Example 4 composition

| | |
|---|---|
| Cyclohexylglycine | 3.0% by weight |
| Cosmetic alcohol | 50.0 |
| Purified water | Balance |

The result was that Example 4 application group of the present invention showed a mean score of 3.9, whereas Comparative Example 1 application group showed a mean score of 2.3. As can be seen from the result, reduction in wrinkles was markedly promoted. This confirmed that the cyclohexylglycine solution has an effect of improving wrinkles of the skin formed by UV rays.

### Wrinkle-improving test on hairless mice (5)

Using the following sample, a wrinkle-improving test was conducted in a manner similar to that described above. Comparative Example 1 was the same as the one described above.

### Example 5 composition

| | |
|---|---|
| Benzenesulfonylglycine | 3.0% by weight |
| Cosmetic alcohol | 50.0 |
| Purified water | Balance |

The result was that Example 5 application group of the present invention showed a mean score of 3.9, whereas Comparative Example 1 application group showed a mean score of 2.7. As can be seen from the result, reduction in wrinkles was markedly promoted. This confirmed that the benzenesulfonylglycine solution has an effect of improving wrinkles of the skin formed by UV rays. Wrinkle-improving test on hairless mice (6)

Using the following sample, a wrinkle-improving test was conducted in a manner similar to that described above. Comparative Example 1 was the same as the one described above.

### Example 6 composition

| | |
|---|---|
| Benzenesulfonylsarcosine | 3.0% by weight |
| Cosmetic alcohol | 50.0 |
| Purified water | Balance |

The result was that Example 6 application group of the present invention showed a mean score of 3.7, whereas Comparative Example 1 application group showed a mean score of 2.4. As can be seen from the result, reduction in wrinkles was markedly promoted. This confirmed that the benzenesulfonylsarcosine solution has an effect of improving wrinkles of the skin formed by UV rays.

### Wrinkle-improving test on hairless mice (7)

Using the following sample, a wrinkle-improving test was conducted in a manner similar to that described above. Comparative Example 1 was the same as the one described above.

### Example 7 composition

| | |
|---|---|
| Benzoyl-L-alanine | 3.0% by weight |
| Cosmetic alcohol | 50.0 |
| Purified water | Balance |

The result was that Example 7 application group of the present invention showed a mean score of 4.0, whereas Comparative Example 1 application group showed a mean score of 2.8. As can be seen from the result, reduction in wrinkles was markedly promoted. This confirmed that the benzoyl-L-alanine solution has an effect of improving wrinkles of the skin formed by UV rays.

### Wrinkle-improving test on hairless mice (8)

Using the following sample, a wrinkle-improving test was conducted in a manner similar to that described above. Comparative Example 1 was the same as the one described above.

### Example 8 composition

| | |
|---|---|
| Piperidine acetate | 3.0% by weight |
| Cosmetic alcohol | 50.0 |
| Purified water | Balance |

The result was that Example 8 application group of the present invention showed a mean score of 3.8, whereas Comparative Example 1 application group showed a mean score of 2.5. As can be seen from the result, reduction in wrinkles was markedly promoted. This confirmed that the piperidine acetate solution has an effect of improving wrinkles of the skin formed by UV rays.

### Wrinkle-improving test on hairless mice (9)

Using the following sample, a wrinkle-improving test was conducted in a manner similar to that described above. Comparative Example 1 was the same as the one described above.

### Example 9 composition

| | |
|---|---|
| Pyrrolidine acetate | 3.0% by weight |
| Cosmetic alcohol | 50.0 |
| Purified water | Balance |

The result was that Example 9 application group of the present invention showed a mean score of 3.9, whereas Comparative Example 1 application group showed a mean score of 2.6. As can be seen from the result, reduction in wrinkles was markedly promoted. This confirmed that the pyrrolidine acetate solution has an effect of improving wrinkles of the skin formed by UV rays.

### Wrinkle-improving test on hairless mice (10)

Using the following sample, a wrinkle-improving test was conducted in a manner similar to that described above. Comparative Example 1 was the same as the one described above.

### Example 10 composition

| | |
|---|---|
| Morpholine acetate | 3.0% by weight |
| Cosmetic alcohol | 50.0 |
| Purified water | Balance |

The result was that Example 10 application group of the present invention showed a mean score of 3.9, whereas Comparative Example 1 application group showed a mean score of 2.7. As can be seen from the result, reduction in wrinkles was markedly promoted. This confirmed that the morpholine acetate solution has an effect of improving wrinkles of the skin formed by UV rays.

### Wrinkle-improving test on hairless mice (11)

Using the following sample, a wrinkle-improving test was conducted in a manner similar to that described above. Comparative Example 1 was the same as the one described above.

### Example 11 composition

| | |
|---|---|
| Cyclohexyl-DL-alanine | 3.0% by weight |
| Cosmetic alcohol | 50.0 |
| Purified water | Balance |

The result was that Example 11 application group of the present invention showed a mean score of 4.0, whereas Comparative Example 1 application group showed a mean score of 2.7. As can be seen from the result, reduction in wrinkles was markedly promoted. This confirmed that the cyclohexyl-DL-alanine solution has an effect of improving wrinkles of the skin formed by UV rays.

### Wrinkle-improving test on hairless mice (12)

Using the following sample, a wrinkle-improving test was conducted in a manner similar to that described above. Comparative Example 1 was the same as the one described above.

### Example 12 composition

| | |
|---|---|
| Carbamoyl-L-alanine | 3.0% by weight |
| Cosmetic alcohol | 50.0 |
| Purified water | Balance |

The result was that Example 12 application group of the present invention showed a mean score of 3.8, whereas Comparative Example 1 application group showed a mean score of 2.6. As can be seen from the result, reduction in wrinkles was markedly promoted. This confirmed that the carbamoyl-L-alanine solution has an effect of improving wrinkles of the skin formed by UV rays. Wrinkle-improving test on hairless mice (13)
Using the following sample, a wrinkle-improving test was conducted in a manner similar to that described above. Comparative Example 1 was the same as the one described above.

### Example 13 composition

| | |
|---|---|
| Aminoglycine | 3.0% by weight |
| Cosmetic alcohol | 50.0 |
| Purified water | Balance |

The result was that Example 13 application group of the present invention showed a mean score of 4.0, whereas Comparative Example 1 application group showed a mean score of 2.8. As can be seen from the result, reduction in wrinkles was markedly promoted. This confirmed that the aminoglycine solution has an effect of improving wrinkles of the skin formed by UV rays.

### Wrinkle-improving test on hairless mice (14)

Using the following sample, a wrinkle-improving test was conducted in a manner similar to that described above. Comparative Example 1 was the same as the one described above.

### Example 14 composition

| | |
|---|---|
| Amidino-L-alanine | 3.0% by weight |
| Cosmetic alcohol | 50.0 |
| Purified water | Balance |

The result was that Example 14 application group of the present invention showed a mean score of 3.8, whereas Comparative Example 1 application group showed a mean score of 2.4. As can be seen from the result, reduction in wrinkles was markedly promoted. This confirmed that the amidino-L-alanine solution has an effect of improving wrinkles of the skin formed by UV rays.

### Wrinkle-improving test on hairless mice (15)

Using the following sample, a wrinkle-improving test was conducted in a manner similar to that described above. Comparative Example 1 was the same as the one described above.

### Example 15 composition

| | |
|---|---|
| 4-methoxybenzoylglycine | 3.0% by weight |
| Cosmetic alcohol | 50.0 |
| Purified water | Balance |

The result was that Example 15 application group of the present invention showed a mean score of 3.7, whereas Comparative Example 1 application group showed a mean score of 2.5. As can be seen from the result, reduction in wrinkles was markedly promoted. This confirmed that the 4-methoxybenzoylglycine solution has an effect of improving wrinkles of the skin formed by UV rays.

Hereinbelow, other examples of wrinkle-improving cosmetics of the present invention are shown.

### A test on improving wrinkles at the corners of the eye

(Method) On the face of a much wrinkled normal healthy male panelist, a lotion of Example 16 or Comparative Example 2 having the following composition was applied three times per day by the half face method (blind test).

### Example 16 lotion

| | |
|---|---|
| Sarcosine | 0.7% by weight |
| Cosmetic alcohol | 15.0 |
| Purified water | Balance |

### Comparative Example 2 lotion

| | |
|---|---|
| Cosmetic alcohol | 15.0% by weight |
| Purified water | Balance |

A replica was obtained from the applied region of the corners of the eye using SILFLO (Flexico Development Ltd.), and the rate of change in the ratio of the wrinkle area before application (0 M), and one month (1 M) and two months (2 M) after application. The ratio of the wrinkle area was determined by analyzing with a wrinkle measuring instrument according to a laser cutting method (Japanese Unexamined Patent Publication (Kokai) No. 7-113623).

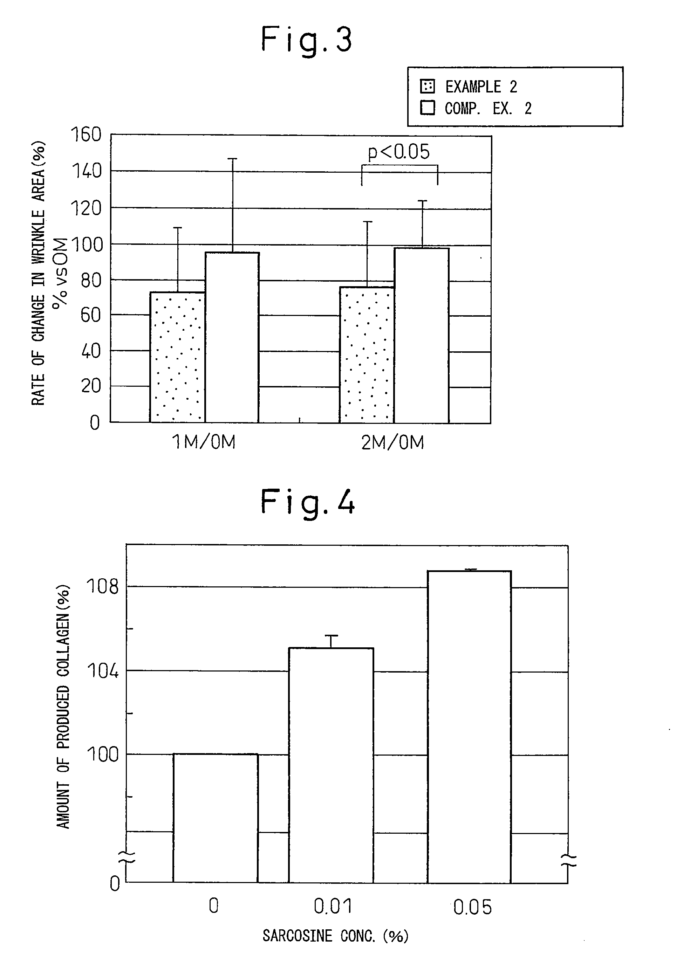

For the ratio of the wrinkle area before application, and one month and two months after application, means of the rate of change with setting the ratio before application at 100% are shown in Fig. 3. The rate of change in the ratio of the wrinkle area one month after application is expressed in 1M/0M and that two months after application is expressed in 2M/0M. In the Comparative Example 2 application side, there was very little change in the wrinkle area compared to that before application, whereas the Example 16 application side exhibited a reduction to about 80% or less of that before application, indicating a significant difference with a significance level of less than 5% in the between-group comparison between Comparative Example 2 and Example 16 two months after composition (Student's paired t-test). This confirmed a significant effect of improving wrinkles in Example 16.

### Examples 17 to 32, Comparative Example 3: wrinkle-improving creams

### A (oil phase)

| | |
|---|---|
| Squalane | 15.0% by weight |
| Vaseline | 7.0 |
| Cetanol | 4.0 |
| Isopropyl myristate | 8.0 |
| Stearic acid monoglycerin ester | 2.0 |
| POE(20) sorbitan monostearate | 2.0 |
| Vitamin E acetate | 1.0 |
| Ethyl paraben | 0.2 |
| Perfume | 0.2 |

### B (Aqueous phase)

| | |
|---|---|
| Glycerin | 4.0 |
| Dipropylene glycol | 4.0 |
| Wrinkle-improving composition (described in the following Table 2) | Described in the following Table 2 |
| Sodium lactate | 2.0 |
| Trisodium edetate | 0.01 |
| Potassium hydroxide | 0.2 |
| Purified water | Balance |
| (Preparation method) A (oil phase) and B (aqueous phase) are each heated to dissolve completely. A is added to B and emulsified with an emulsifier. The emulsified product was cooled with a heat exchanger to yield a cream. | |

**Table 2**

| Wrinkle-improving agent (compound name) | | Amount blended wt% |
|---|---|---|
| Ex. 17 | Sarcosine | 1.0 |
| Ex. 18 | Sarcosine | 0.5 |
| Ex. 19 | Sarcosine | 0.05 |
| Ex. 20 | Sarcosine | 0.01 |
| Ex. 21 | Ethylglycine | 3.0 |
| Ex. 22 | Ethylglycine | 1.0 |
| Ex. 23 | Ethylglycine | 0.1 |
| Ex. 24 | Ethylglycine | 0.05 |
| Ex. 25 | Benzoyl-DL-serine | 3.0 |
| Ex. 26 | Benzyloxycarbonyl-L-serine | 3.0 |
| Ex. 27 | Cyclohexylglycine | 3.0 |
| Ex. 28 | Benzensulfonyl sarcosine | 2.0 |
| Ex. 29 | Aminoglycine | 3.0 |
| Ex. 30 | Nicotinoylglycine | 1.0 |
| Ex. 31 | N-carbamoyl-L-alanine | 3.0 |
| Ex. 32 | N-acetyl-L-alanine | 3.0 |
| Comp. Ex. 3 | Not blended | |

### "Test on human wrinkle-improving effect"

(Testing method) On the wrinkle-improving and fine wrinkle-improving effects of the creams of the above Examples and Comparative Examples, the test was carried out as follows. One hundred eighty female subjects aged 40 to 60's were divided into nine groups of 20 persons per group, and the creams of the above Examples and Comparative Examples were used on the corners of their eyes for two consecutive months. Cosmetic technicians conducted the visual evaluation of wrinkles and fine wrinkles, and assessed in four grades: markedly effective, effective, slightly effective and not effective, and judged the effect according to the following criteria:
Markedly effective: Wrinkles and fine wrinkles became hardly noticeable.
Effective: Wrinkles and fine wrinkles became slightly noticeable.
Slightly effective: Wrinkles and fine wrinkles became less noticeable than before the test.
Not effective: No changes observed.

### (Judgment)

Excellent: The ratio of persons who proved markedly effective, effective, or slightly effective combined among all the subjects is 80% or greater.
Good: The ratio of persons who proved markedly effective, effective, or slightly effective combined among all the subjects is 50% or greater and less than 80%.
Passable: The ratio of persons who proved markedly effective, effective, or slightly effective combined among all the subjects is 30% or greater and less than 50%.
Not passable: The ratio of persons who proved markedly effective, effective, or slightly effective combined among all the subjects is less than 30%.

**Table 3**

| | Wrinkle-improving effect |
|---|---|
| Example 17: | Excellent |
| Example 18: | Excellent |
| Example 19: | Excellent |
| Example 20: | Good |
| Example 21: | Good |
| Example 22: | Good |
| Example 23: | Good |
| Example 24: | Passable |
| Example 25: | Excellent |
| Example 26: | Excellent |
| Example 27: | Excellent |
| Example 28: | Excellent |
| Example 29: | Excellent |
| Example 30: | Excellent |
| Example 31: | Excellent |
| Example 32: | Excellent |
| Comparative Example 3: | Not passable |

As can be seen from "Table 3", the wrinkle-improving cosmetics of the present invention have an excellent improving effect.

### Example 33

The effect of the α-amino acid derivative of the present invention on the amount produced of collagen was tested.

The production of collagen was measured in the following method. Fibroblasts were plated on 24 wells, and were allowed to stand while culturing until the cells fully adhered. Then, the culture liquid (DMEM supplemented with growth factors) was replaced with a medium (DMEM without growth factors) to which the drug (sarcosine) was added to the desired concentration, and cultured for two days. The culture supernatant was removed, and the production amount of type I collagen was determined using the TAKARA's Procollagen type I-Cppeptide EIA KIT. Cell counts were determined by measuring the amount of DNA using Hoechst and then converting it to a cell count. Finally the production amount of collagen was compared as the production amount of collagen per unit cell. The result is shown in Fig. 4.

It was demonstrated that the ability of cells to produce collagen increases with sarcosine addition in dose dependent manner.

### Formulation Example 1: Wrinkle-improving cream

| | |
|---|---|
| Stearic acid | 5.0% by weight |
| Stearyl alcohol | 4.0 |
| Isopropyl myristate | 18.0 |
| Glycerin monostearic acid ester | 3.0 |
| Propylene glycol | 10.0 |
| Sarcosine | 20.0 |
| Caustic potash | 0.2 |
| Sodium hydrogensulfite | 0.01 |
| Antiseptic | q.s. |
| Perfume | q.s. |
| Ion-exchanged water | Balance |

(Preparation method) To ion-exchanged water, propylene glycol and caustic potash are added, dissolved, and heated to and kept at 70°C (aqueous phase). The other ingredients are mixed, melted, and kept at 70°C (oil phase). The oil phase is gradually added to the aqueous phase, and after the entire phase has been added, it is kept at the temperature and allowed to react. Then, it was emulsified to homogeneity with a homogenizer, and cooled while stirring well to 30°C for preparation.

### Formulation Example 2: Wrinkle-improving cream

| | |
|---|---|
| Stearic acid | 6.0% by weight |
| Sorbitan monostearic acid ester | 2.0 |
| Polyoxyethylene (20 mole) sorbitan monostearic acid ester | 1.5 |
| Propylene glycol | 10.0 |
| Sarcosine | 7.0 |
| Glycerin trioctanoate | 10.0 |
| Squalene | 5.0 |
| Sodium hydrogensulfite | 0.01 |
| Ethylparabene | 0.3 |
| Perfume | q.s. |
| Ion-exchanged water | Balance |

(Preparation method) To ion-exchanged water, propylene glycol is added, dissolved, and heated to and kept at 70°C (aqueous phase). The other ingredients are mixed, melted, and kept at 70°C (oil phase). The oil phase is added to the aqueous phase for preparatory emulsification, and after it was emulsified to homogeneity with a homogenizer, it is cooled while stirring well to 30°C for preparation.

### Formulation Example 3: Wrinkle-improving cream

| | |
|---|---|
| Stearyl alcohol | 7.0% by weight |
| Stearic acid | 2.0 |
| Hydrogenated lanolin | 2.0 |
| Squalane | 5.0 |
| 2-octyldodecyl alcohol | 6.0 |
| Polyoxyethylene (25 mole) cetyl alcohol ether | 3.0 |
| Glycerin monostearic acid ester | 2.0 |
| Propylene glycol | 5.0 |
| Sarcosine | 0.001 |
| Perfume | q.s. |
| Sodium hydrogensulfite | 0.03 |
| Ethylparabene | 0.3 |
| Ion-exchanged water | Balance |

(Preparation method) To ion-exchanged water, propylene glycol is added, dissolved, and heated to and kept at 70°C (aqueous phase). The other ingredients are mixed, melted, and kept at 70°C (oil phase). The oil phase is added to the aqueous phase for preparatory emulsification, and after it is emulsified to homogeneity with a homogenizer, it is cooled while stirring well to 30°C for preparation.

### Formulation Example 4: Wrinkle-improving milky lotion

| | |
|---|---|
| Stearic acid | 2.5% by weight |
| Cetyl alcohol | 1.5 |
| Vaseline | 5.0 |
| Liquid paraffin | 10.0 |
| Polyoxyethylene (10 mole) monooleic acid ester | 2.0 |
| Polyethylene glycol 1500 | 3.0 |
| Triethylamine | 1.0 |
| Sarcosine | 10.0 |
| Sodium hydrogensulfite | 0.01 |
| Ethylparabene | 0.3 |
| Carboxyvinyl polymer | 0.05 |
| Perfume | q.s. |
| Ion-exchanged water | Balance |

(Preparation method) To a small amount of ion-exchanged water, carboxyvinyl polymer is dissolved (phase A). To the rest of ion-exchanged water, polyethylene glycol 1500 and triethylamine are added, heat-dissolved and kept at 70°C (oil phase). The other ingredients are mixed, melted, and kept at 70°C (oil phase). The oil phase is added to the aqueous phase for preparatory emulsification, and phase A is added thereto and emulsified to homogeneity with a homogenizer, it is cooled while stirring well to 30°C for preparation.

### Formulation Example 5: Wrinkle-improving milky lotion

| (Oil phase part) | |
|---|---|
| Stearyl alcohol | 1.5% by weight |
| Squalene | 2.0 |
| Vaseline | 2.5 |
| Deodorizing liquid lanolin | 1.5 |
| Primrose oil | 2.0 |
| Isopropyl myristate | 5.0 |
| Glycerin monooleate | 2.0 |
| Polyoxyethylene (60 mole) hydrogenated castor oil | 2.0 |
| Tocopherol acetate | 0.05 |
| Ethylparabene | 0.2 |
| Butylparabene | 0.1 |
| Ethylglycine | 1.0. |
| Sarcosine | 1.0 |
| Perfume | q.s. |
| (Aqueous phase part) Sodium hydrogensulfite | 0.01% by weight |
| Glycerin | 5.0 |
| hyaluronate sodium | 0.01 |
| Carboxyvinyl polymer | 0.2 |
| Potassium hydroxide | 0.2 |
| Ion-exchanged water | Balance |

(Preparation method) The oil phase part is dissolved at 70°C. The aqueous phase part is dissolved at 70°C, and the oil phase part is mixed to the aqueous phase part, which is emulsified with an emulsifier, and then cooled with a heat exchanger to 30°C for preparation.

### Formulation Example 6: Wrinkle-improving gel

| | |
|---|---|
| 95% Ethyl alcohol | 10.0% by weight |
| Dipropylene glycol | 15.0 |
| Polyoxyethylene (50 mole) oleyl alcohol ether | 2.0 |
| Carboxyvinyl polymer | 1.0 |
| Caustic soda | 0.15 |
| L-Arginine | 0.1 |
| Sarcosine | 1.0 |
| Methylparabene | 0.2 |
| Perfume | q.s. |
| Ion-exchanged water | Balance |

(Preparation method) To ion-exchanged water, the carboxyvinyl polymer is dissolved to homogeneity, and, on the other hand, in 95% ethanol, sarcosine and polyoxyethylene (50 mole) oleyl alcohol ether are dissolved, and added to the aqueous phase. Then, after adding the other ingredients, it is neutralized with caustic soda and L-arginine, and thickened for preparation.

### Formulation Example 7: Wrinkle-improving essense

| (Phase A) | |
|---|---|
| Ethanol (95%) | 10.0% by weight |
| Polyoxyethylene (20 mole) octyldodecanol | |
| | 1.0 |
| Methylparabene | 0.15 |
| Pantothenil ethyl ether | 0.1 |
| Sarcosine | 0.05 |

| (Phase B) | |
|---|---|
| Potassium hydroxide | 0.1 |

| (Phase C) | |
|---|---|
| Glycerin | 5.0 |
| Dipropylene glycol | 10.0 |
| Sodium hydrogensulfite | 0.03 |
| Carboxyvinyl polymer | 0.2 |
| Purified water | Balance |

(Preparation method) Phase A and Phase C are each dissolved to homogeneity, and Phase A is added to Phase C, which is then solubilized. Then, after adding Phase B, it is filled for preparation.

### Formulation Example 8: Wrinkle-improving pack

| (Phase A) | |
|---|---|
| Dipropylene glycol | 5.0% by weight |
| Polyoxyethylene (60 mole) hydrogenated castor oil | 5.0 |

| (Phase B) | |
|---|---|
| Sarcosine | 1.0 |
| Olive oil | 5.0 |
| Tocopherol acetate | 0.2 |
| Eethylparabene | 0.2 |
| Perfume | 0.2 |

| (Phase C) | |
|---|---|
| Sodium hydrogensulfite | 0.03 |
| Polyvinyl alcohol (degree of saponification 90, degree of polymerization 2000) | 13.0 |
| Ethanol | 7.0 |
| Purified water | Balance |

(Preparation method) Phase A, Phase B and Phase C are each dissolved to homogeneity, and Phase B is added to Phase A, which is then solubilized. Then, after adding Phase C thereto, it is filled for preparation.

### Formulation Example 9: Wrinkle-improving ointment

| | |
|---|---|
| Polyoxyethylene (30 mole) cetyl ether | 2.0% by weight |
| Glycerin monostearate | 10.0 |
| Liquid paraffin | 10.0 |
| Vaseline | 40.0 |
| Cetanol | 6.0 |
| Methylparabene | 0.1 |
| Butylparabene | 0.1 |
| Glycerin monostearic acid ester | 2.0 |
| Sarcosine | 5.0 |
| Propylene glycol | 10.0 |
| Ion-exchanged water | Balance |
| Perfume | q.s |

(Preparation method) To ion-exchanged water, propylene glycol is added, dissolved and heated to and kept at 70°C (aqueous phase). The other ingredients are mixed and dissolved at 70°C (oil phase). To the above aqueous phase, the oil phase is added, homogeneously emulsified with an emulsifier, and after cooling it is filled for preparation.

### Formulation Example 10: Wrinkle-improving cream

| | |
|---|---|
| Liquid paraffin | 8% by weight |
| Vaseline | 3 |
| Dimethylpolysiloxane | 2 |
| Stearyl alcohol | 3 |
| Behenyl alcohol | 2 |
| Glycerin | 5 |
| Dipropylene glycol | 4 |
| Trehalose | 1 |
| Tetra 2-ethyl hexanoic acid pentaerythrite | 4 |
| Monoisostearic acid polyoxyethylene glyceryl | 2 |
| Monostearic acid polyoxyethylene glycerin | 1 |
| Lipophilic monostearic acid glycerin | 2 |
| Citric acid | 0.05 |
| Sodium citrate | 0.05 |
| Potassium hydroxide | 0.015 |
| Oil-soluble Licorice extract | 0.1 |
| Retinol palmitate (1 million units) | 0.25 |
| Sarcosine | 1.0 |
| Tocopherol acetate | 0.1 |
| Paraoxybenzoic acid ester | q.s. |
| Phenoxyethanol | q.s. |
| Dibutylhydroxy toluene | q.s. |
| Trisodium edetate | 0.05 |
| 4-t-butyl-4'-methoxydibenzoyl methane | 0.01 |
| Paramethoxy cinnamic acid 2-ethyl hexyl | 0.1 |
| β-carotene | 0.01 |
| Polyvinyl alcohol | 0.5 |
| Hydroxyethyl cellulose | 0.5 |
| Carboxyvinyl polymer | 0.05 |
| Purified water | Balance |
| Perfume | q.s. |

### Formulation Example 11: Wrinkle-improving cream

| | |
|---|---|
| Vaseline | 2% by weight |
| Dimethylpolysiloxane | 2 |
| Ethanol | 5 |
| Behenyl alcohol | 0.5 |
| Batyl alcohol | 0.2 |
| Glycerin | 7 |
| 1,3-butylene glycol | 5 |
| Polyethylene glycol 20000 | 0.5 |
| Jojoba oil | 3 |
| Squalane | 2 |
| Hydroxystearic acid phitosteryl | 0.5 |
| Tetra 2-ethyl hexanoic acid pentaerythrite | 1 |
| Polyoxyethylene hydrogenated castor oil | 1 |
| Potassium hydroxide | 0.1 |
| Sodium pyrosulfife | 0.01 |
| Sodium hexametaphosphate | 0.05 |
| Glycyrrhetinic acid stearyl | 0.1 |
| Pantothenyl ethyl ether | 0.1 |
| Arbutin | 7 |
| Tranexamic acid methylamide hydrochloride | 11 |
| Sarcosine | 1 |
| Tocopherol acetate | 0.1 |
| Sodium hyaluronate | 0.05 |
| Paraoxybenzoic acid ester | q.s. |
| Trisodium edetate | 0.05 |
| 4-t-butyl-4'-methoxydibenzoyl methane | 0.1 |
| Diparamethoxy cinnamic acid mono-2-ethyl hexanoic acid glyceryl | 0.1 |
| Yellow iron oxide | q.s. |
| Xanthan gum | 0.1 |
| Carboxyvinyl polymer | 0.2 |
| Purified water | Balance |

### Formulation Example 12: Wrinkle-improving cream

| | |
|---|---|
| Vaseline | 2% by weight |
| Dimethylpolysiloxane | 2 |
| Ethanol | 5 |
| POE14POP7 dimethylether | 1.0 |
| Behenyl alcohol | 0.5 |
| Batyl alcohol | 0.2 |
| Glycerin | 7 |
| 1,3-butylene glycol | 5 |
| Polyethylene glycol 20000 | 0.5 |
| Jojoba oil | 3 |
| Squalane | 2 |
| Hydroxystearic acid phitosteryl | 0.5 |
| Tetra 2-ethyl hexanoic acid pentaerythrite | 1 |
| Polyoxyethylene hydrogenated castor oil | 1 |
| Potassium hydroxide | 0.1 |
| Sodium pyrosulfife | 0.01 |
| Sodium hexametaphosphate | 0.05 |
| Glycyrrhetinic acid stearyl | 0.1 |
| Pantothenyl ethyl ether | 0.1 |
| Arbutin | 7 |
| Glycylglycine | 1.0 |
| Soy bean resorcin | 0.1 |
| Hydrolyzing yeast extract | 0.1 |
| Tranexamic acid methylamide hydrochloride | 11 |
| Benzoyl-DL-serine | 1 |
| Tocopherol acetate | 0.1 |
| Sodium hyaluronate | 0.05 |
| Paraoxybenzoic acid ester | q.s. |
| Trisodium edetate | 0.05 |
| 4-t-butyl-4'-methoxydibenzoyl methane | 0.1 |

Diparamethoxy cinnamic acid mono-2-ethyl hexanoic acid glyceryl

| | |
|---|---|
| Yellow iron oxide | q.s. |
| Xanthan gum | 0.1 |
| Carboxyvinyl polymer | 0.2 |
| Purified water | Balance |

### Formulation Example 13: Wrinkle-improving cream

| | |
|---|---|
| Liquid paraffin | 8% by weight |
| Vaseline | 3 |
| Dimethylpolysiloxane | 2 |
| Stearyl alcohol | 3 |
| Behenyl alcohol | 2 |
| Glycerin | 5 |
| Dipropylene glycol | 4 |
| Trehalose | 1 |
| Glycylglycine | 1.6 |
| Tetra 2-ethyl hexanoic acid pentaerythrite | 4 |
| Monoisostearic acid polyoxyethylene glyceryl | 2 |
| Monostearic acid polyoxyethylene glycerin | 1 |
| Lipophilic monostearic acid glycerin | 2 |
| Citric acid | 0.05 |
| Sodium citrate | 0.05 |
| Potassium hydroxide | 0.015 |
| Oil-soluble Licorice extract | 0.1 |
| Retinol | 0.25 |
| Benzyloxycarbonyl-L-serine | 3.0 |
| Tocopherol acetate | 0.1 |
| Paraoxybenzoic acid ester | q.s. |
| Phenoxyethanol | q.s. |
| Dibutylhydroxy toluene | q.s. |
| Trisodium edetate | 0.05 |
| 4-t-butyl-4'-methoxydibenzoyl methane | 0.01 |
| Paramethoxy cinnamic acid 2-ethyl hexyl | 0.1 |
| POE14POP7 dimethylether | 2.0 |
| β-carotene | 0.01 |
| Polyvinyl alcohol | 0.5 |
| Hydroxyethyl cellulose | 0.5 |
| Carboxyvinyl polymer | 0.05 |
| Purified water | Balance |
| Perfume | q.s. |

### Formulation Example 14: Wrinkle-improving essense

| | |
|---|---|
| Ethanol (95%) | 10.0% by weight |
| Polyoxyethylene (20 mole) octyldodecanol | 1.0 |
| Methylparabene | 0.15 |
| Pantothenil ethyl ether | 0.1 |
| Amidinoglycine (guanidino acetic acid) | 1.0 |
| Potassium hydroxide | 0.1 |
| Glycylglycine | 2.0 |
| Mangosteen extract | 0.1 |
| Ononis extract | 0.1 |
| Beech bud extract | 0.1 |
| Glycerin | 5.0 |
| POE14POP7 dimethyl ether | 1.0 |
| Dipropylene glycol | 10.0 |
| Sodium hydrogensulfite | 0.03 |
| Carboxyvinyl polymer | 0.2 |
| Purified water | Balance |

### Formulation Example 15: Wrinkle-improving essense

| | |
|---|---|
| Ethanol (95%) | 10.0% by weight |
| Polyoxyethylene (20 mole) octyldodecanol | 1.0 |
| Methylparabene | 0.15 |
| Pantothenil ethyl ether | 0.1 |
| Glycylglycine | 3.0 |
| Bupleuri radix extract | 0.1 |
| Sophorae radix extract | 0.1 |
| Citrus junos seed extract | 0.1 |
| Cyclohexyl-DL-alanine | 1.0 |
| Potassium hydroxide | 0.1 |
| Glycerin | 5.0 |
| POE14POP7 dimethyl ether | 1.0 |
| Dipropylene glycol | 10.0 |
| Sodium hydrogensulfite | 0.03 |
| Carboxyvinyl polymer | 0.2 |
| Purified water | Balance |

Any of the wrinkle-improving cosmetics obtained in Formulation Examples 1 to 15 exhibited wrinkle-improving effects in the test on wrinkle-improving effect similar to those conducted in Examples 17 to 32.

## Claims

1. A wrinkle-preventing and improving composition comprising one or more than one compound selected from the group consisting of α-amino acid derivatives represented by the following general formula (1) and salts thereof: 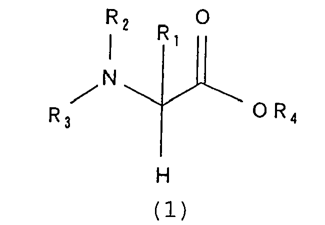 wherein, R₁ represents hydrogen atom, CH₃ group or CH₂OH group,
R₂ and R₃ each independently represent hydrogen atom, alkyl group or alkenyl group having 1 to 4 carbons, acyl group having 2 to 6 carbons, carbamoyl group, amidino group, pyridylcarbonyl group, benzyloxycarbonyl group, cyclohexyl group, cyclohexanecarbonyl group, benzoyl group, benzenesulfonyl group, phenyl group, or benzyl group, provided that R₂ and R₃ cannot be hydrogen atom at the same time, or
R₂ and R₃ together with N atom to which they are bound may form a ring structure having a total carbon number of 4 to 6, in which case said ring structure may optionally contain oxygen atom as a heteroatom,
R₄ represents hydrogen atom, alkyl group or alkenyl group having 1 to 18 carbons, phenyl group, or benzyl group,
wherein the cyclohexyl moiety or the phenyl moiety of R₂, R₃ and R₄, or the ring structure containing N atom formed by R₂ and R₃ may optionally have alkyl group having 1 to 3 carbons, alkoxyl group having 1 to 3 carbons, or hydroxyl group,
or, when R₁ and R₄ are hydrogen atom, it cannot be that one of R₂ and R₃ is benzyloxycarbonyl group and the other is hydrogen atom, nor that one of R₂ and R₃ is amidino group and the other is methyl group.

2. The wrinkle-preventing and improving composition according to claim 1 wherein said α-amino acid derivative is sarcosine or benzoyl-DL-serine or a salt thereof.
